# EUROPÄISCHE PATENTANMELDUNG

(11) **EP 2 193 756 A1**
(43) Veröffentlichungstag der Anmeldung: **09.06.2010**
(21) Anmeldenummer: 09015057.4
(22) Anmeldetag: 04.12.2009
(51) Int. Cl.: A61C 1/08

(54) **Führungsschiene und Schlitten für einen Knochenbohrer**

(30) Priorität: 04.12.2008 DE 102008060521
(71) Anmelder: Stauder, Alexander, 90449 Nürnberg (DE)
(72) Erfinder: Stauder, Alexander, 90449 Nürnberg (DE)
(74) Vertreter: Keilitz, Wolfgang

(57) **Zusammenfassung**

Die Erfindung betrifft eine Führungsschiene (1) und einen zugehörigen Schlitten (11) für einen Knochenbohrer. Die Führungsschiene (1) umfasst ein Führungselement (2) zum Führen des Schlittens (11) in einer geplanten Bohrachse (19), Befestigungsmittel (3) zum Befestigen der Führungsschiene an einem anderen Element (8), sowie Mittel (4) Abhalten des oralen mukoperiostalen Lappens im Mundraum des Patienten.

## Beschreibung

### Stand der Technik

Die Erfindung betrifft eine Führungsschiene für einen Knochenbohrer gemäß dem Oberbegriff des Patentanspruchs 1, sowie einen Schlitten für einen solchen Knochenbohrer gemäß dem Oberbegriff des Patentanspruchs 10.

Beim Setzen von Dental-Implantaten müssen Bohrtiefe und Richtung der Implantatbohrung sehr genau eingehalten werden. Andernfalls können benachbarte Zahnwurzeln oder angrenzende anatomische Strukturen verletzt werden. Bei der prä-operativen Planung einer Implantation wird üblicherweise eine chirurgische Schablone hergestellt, die an der zuvor festgelegten Implantationsstelle eine Bohrführungshülse aufweist. Diese Schablone wird dem Patienten für den operativen Eingriff in den Mund eingesetzt. Beim Bohren der Implantatbohrung wird der Bohrer von der Bohrführungshülse geführt. Ein Problem dieser Schablonen besteht jedoch darin, dass die Bohrführungshülsen beim Bohren in direkten Kontakt mit dem Bohrer kommen und die Bohrerkühlung behindern. Darüber hinaus sind diese Schablonen an der Implantationsstelle vollständig geschlossen, wodurch die OP-Sicht behindert wird und die Bohrung blind durchgeführt werden muss. Ein weiteres Problem besteht darin, dass der Bohrer von oben in die Führungshülse eingesetzt werden muss, was gerade im hinteren Bereich des Mundraums schwierig ist.

### Offenbarung der Erfindung

Es ist somit die Aufgabe der vorliegenden Erfindung, eine Vorrichtung zur Durchführung einer Implantatbohrung zu schaffen, die einfach zu handhaben und kostengünstig ist.

Gelöst wird diese Aufgabe gemäß der Erfindung durch die im Patentanspruch 1 sowie im Patentanspruch 10 angegebenen Merkmale. Weitere Ausgestaltungen der Erfindung sind Gegenstand von Unteransprüchen.

Ein wesentlicher Aspekt der Erfindung besteht darin, eine Führungsschiene für einen Knochenbohrer vorzusehen, die einen Schlitten, an dem das Handstück des Knochenbohrers befestigt wird, entlang der geplanten Bohrrichtung führt. Die Führungsschiene umfasst im Wesentlichen ein Führungselement zum Führen des Schlittens in der geplanten Bohrrichtung, Befestigungsmittel zum Befestigen der Führungsschiene an einem anderen Element, wie z.B. an einer Schablone, und vorzugsweise auch Mittel zum Abhalten oraler mukoperiostaler Lappen während der OP. Die erfindungsgemäße Führungsschiene kann im Mundraum des Patienten sehr genau ausgerichtet werden und den Knochenbohrer exakt in der geplanten Bohrrichtung führen. Darüber hinaus ermöglicht sie die Verwendung einer an der Implantationsstelle offenen Schablone, wodurch eine direkte Sicht auf das OP-Feld möglich wird. Eine Kühlung des Bohrers und Absaugung von Flüssigkeit in unmittelbarer Nähe der Implantationsstelle sind damit ebenfalls möglich.

Das Führungselement der Führungsschiene ist vorzugsweise ein lang gestrecktes, stabförmiges Element. Die Längsrichtung der Führungsschiene entspricht im befestigten Zustand der geplanten Bohrrichtung.

Die genannten Mittel zum Abhalten oraler mukoperiostaler Lappen (im Folgenden Haltemittel) sind vorzugsweise an oder nahe einem Ende der Führungsschiene angeordnet. Gemäß einer bevorzugten Ausführungsform der Erfindung sind die Haltemittel pfotenartig gebildet.

Die Haltemittel erstrecken sich vorzugsweise quer zu einer Längsrichtung der Führungsschiene und ragen seitlich vom Führungselement nach außen.

Die genannten Befestigungsmittel zum Befestigen der Führungsschiene sind vorzugsweise derart ausgebildet, dass sie entweder direkt an der chirurgischen Schablone oder indirekt über ein Zwischenstück an dieser befestigt werden können. Gemäß einer bevorzugten Ausführungsform der Erfindung umfassen die Befestigungsmittel einen Arm, der sich quer zur Längsrichtung des Führungselements erstreckt und von diesem nach außen wegragt.

Die Haltemittel und die Befestigungsmittel erstrecken sich vorzugsweise etwa in entgegengesetzte Richtungen vom Führungselement weg. In Längsrichtung der Führungsschiene sind sie vorzugsweise versetzt zueinander angeordnet.

Die Befestigungsmittel sind vorzugsweise an einem mittleren Abschnitt des Führungselements angeordnet. Die Haltemittel befinden sich vorzugsweise an einem Ende des Führungselements.

Ein zugehöriger Schlitten für einen Knochenbohrer umfasst einen Grundkörper mit einem Kupplungsmittel, das mit der Führungsschiene zusammenwirkt, sowie Befestigungsmittel zur Befestigung des Schlittens am Handstück des Knochenbohrers.

Die genannten Befestigungsmittel erstrecken sich vorzugsweise im Wesentlichen quer zur Führungsachse des Schlittens. Sie sind vorzugsweise derart ausgebildet, dass das Handstück relativ zum Schlitten geschwenkt werden kann.

Gemäß einer bevorzugten Ausführungsform der Erfindung umfassen die Befestigungsmittel einen vom Schlitten-Grundkörper seitlich wegragenden Arm, in dem eine Öffnung zur Aufnahme des eigentlichen Spiralbohrers vorgesehen ist. Der Arm ist vorzugsweise einstückig mit dem Grundkörper gebildet. Die Befestigungsmittel umfassen ferner ein Befestigungselement, das am Handstück des Knochenbohrers befestigt wird. Das Befestigungselement kann beispielsweise als eine Art Schelle ausgebildet sein, die z. B. an einem Schaft oder Griff des Handstücks festgeschraubt werden kann und eine Klemmverbindung herstellt.
Das Befestigungselement umfasst vorzugsweise eine Öffnung, die im montierten Zustand mit der Öffnung des vorstehend beschriebenen Armes fluchtet, so dass das Befestigungselement relativ zum Grundkörper des Schlittens schwenkbar ist. Die Schwenkachse entspricht vorzugsweise der Achse des Bohrers. Die Befestigungsmittel umfassen vorzugsweise kein separates Gelenk.

Das Befestigungselement kann alternativ auch direkt am Handstück fixiert werden. Zu diesem Zweck kann z. B. eine Mutter am Handstück vorgesehen sein. Andere bekannte Befestigungsmittel können genauso verwendet werden.

Gemäß einer speziellen Ausführungsform der Erfindung hat der Schlitten einen verstellbaren Tiefenbegrenzer, mittels dessen die Bohrtiefe eingestellt werden kann. Der Grundkörper des Schlittens umfasst in diesem Fall vorzugsweise wenigstens eine Aufnahme für den Tiefenbegrenzer, in der der Tiefenbegrenzer z. B. gleitend verstellt werden kann. Ferner sind vorzugsweise Mittel, z. B. eine Schraube, zum Fixieren des Tiefenbegrenzers in einer gewünschten Position vorgesehen. Eine Rastverbindung für den Tiefenbegrenzer ist ebenfalls realisierbar.

Die im Schlitten vorgesehene Führung, die mit der Führungsschiene zusammenwirkt, ist vorzugsweise derart ausgebildet, dass sie zumindest in einem Teilabschnitt seitlich zugänglich ist. Dies hat den wesentlichen Vorteil, dass der Schlitten im Wesentlichen quer zur späteren Bohrrichtung auf die Führungsschiene aufgesetzt werden kann. Der Schlitten mit dem daran befestigten Handstück muss also nicht von oben, sondern kann von der Seite auf die Führungsschiene aufgesetzt werden. Dies erleichtert das Aufsetzen des Schlittens wesentlich, insbesondere bei engen Platzverhältnissen und im Seitenzahnbereich.

Gemäß einer bevorzugten Ausführungsform der Erfindung ist die Führung als eine Art Gleitschacht bzw. Führungsrille ausgebildet. Die Führung ist vorzugsweise in wenigstens einem Teilabschnitt derart ausgebildet, dass sie das Führungselement der Führungsschiene vollständig umschließt.

Führungsschiene und Schlitten können natürlich auch umgekehrt ausgebildet sein. D. h. der Gleitschacht könnte z. B. an der Führungsschiene, und das geführte Element am Schlitten vorgesehen sein.

### Kurze Beschreibung der Zeichnungen

Die Erfindung wird nachstehend anhand der beigefügten Zeichnungen beispielhaft näher erläutert. Es zeigen:
- Fig. 1a: eine Seitenansicht einer Führungsschiene gemäß einer bevorzugten Ausführungsform der Erfindung;
- Fig. 1b: ein Zwischenstück zur Befestigung an einer chirurgischen Schablone;
- Fig. 1c: eine Aufsicht auf das Zwischenstück von Fig. 1b mit eingesetzter Führungsschiene von Fig. 1a;
- Fig. 2a: eine Seitenansicht eines Schlittens mit einem daran befestigten Handstück eines Knochenbohrers;
- Fig. 2b: eine um 90° gedrehte Ansicht des Schlittens von Fig. 2a; und
- Fig. 2c: eine Ausführungsform eines Bohrtiefenbegrenzers.

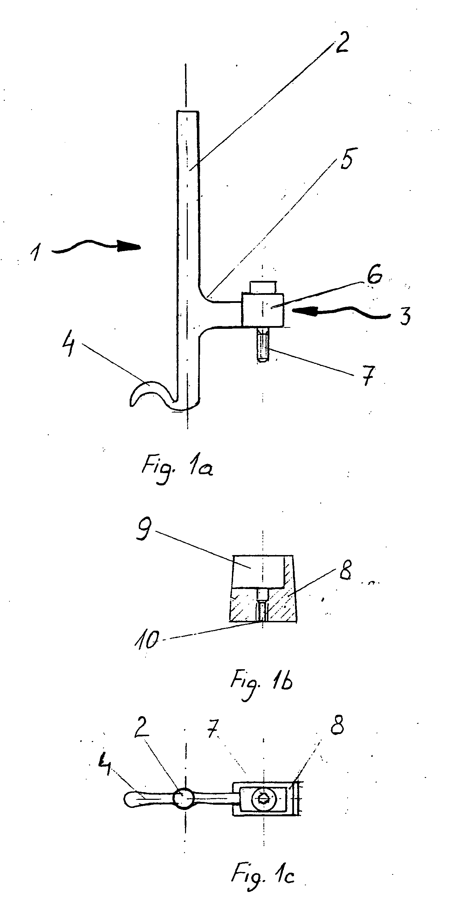

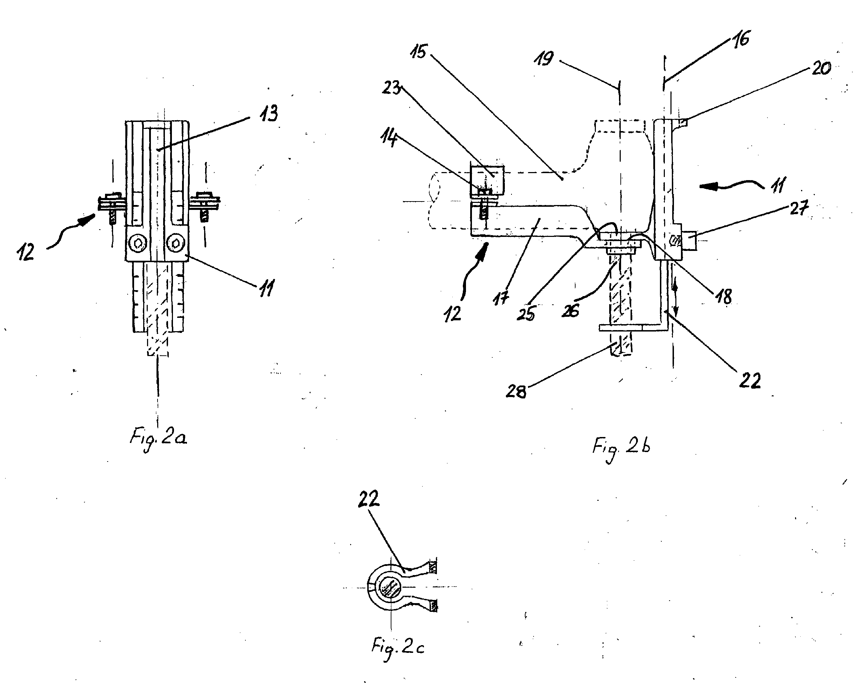

Fig. 1a zeigt eine Seitenansicht einer Führungsschiene 1 zum Führen eines Knochenbohrers während einer Implantatbohrung. Das Handstück 15 (siehe Fig. 2b) des Knochenbohrers ist dabei an einem Schlitten 11 befestigt, wie er in den Fig. 2a und 2b gezeigt ist. Wenn der Schlitten 11 auf der Führungsschiene 1 aufgesetzt ist, wird er, zusammen mit dem Handstück 15, entlang der Längsachse 14 der Führungsschiene 1 geführt. Die Bohrung kann dadurch sehr genau entsprechend der Planung ausgeführt werden.

Die Führungsschiene 1 umfasst im Wesentlichen ein lang gestrecktes, stabförmiges Führungselement 2 zum Führen des Schlittens 11, Befestigungsmittel 3 zum Befestigen der Führungsschiene an einer Schablone (nicht gezeigt) und Haltemittel 4 zum Abhalten des oralen mukoperiostalen Lappens im Mundraum des Patienten. Das Haltemittel 4 ist bei dieser Ausführungsform pfotenartig gebildet und erstreckt sich im Wesentlichen quer zur Längsachse 14 des Führungselements 2. Die Befestigungsmittel 3 umfassen einen seitlich vom Führungselement 2 wegragenden Arm 5, an dessen Ende eine Schraubenaufnahme 6 für eine Schraube 7 vorgesehen ist. Die in der Schraubenaufnahme 6 vorgesehene Öffnung zeigt dabei im Wesentlichen in Längsrichtung der Führungsschiene 1.

Die Befestigungsmittel 3 sind an einem mittleren Abschnitt des Führungselements 2 angeordnet. Das Haltemittel 4 ist dagegen an einem Ende des Führungselements 2 angeordnet. Die Befestigungsmittel 3 und das Haltemittel 4 erstrecken sich im Wesentlichen in entgegengesetzte Richtungen, quer zur Längsrichtung 14.

Fig. 1b zeigt ein Zwischenstück 8 zur Befestigung der Führungsschiene 1. Im anwendungsbereiten Zustand ist ein Zwischenstück 8 in eine Schablone eingebettet. Hierzu umfasst das Zwischenstück 8 vorzugsweise retentive Mittel, wie z.B. einen Vorsprung, die das Zwischenstück 8 im Kunststoff der Schablone verankern. Wie zu erkennen ist, umfasst das Zwischenstück 8 auch eine Aufnahme 9, die an die Größe der Befestigungsmittel 3 angepasst ist. In der Basis des Zwischenstücks 8 ist ferner ein Gewinde 10 vorgesehen, in das die Schraube 7 geschraubt wird.

Fig. 1c zeigt eine Aufsicht auf die Führungsschiene 1 und das Zwischenstück 8 im zusammengesetzten Zustand.

Fig. 2a und 2b zeigen verschiedene seitliche Ansichten eines Schlittens 11, der an die Führungsschiene von Fig. 1a gekoppelt werden kann. Der Schlitten 11 umfasst einen Grundkörper 16 mit einer Führung 13 in Form einer Rille bzw. eines Gleitschachtes. Die Führungsrille 13 ist zum größten Teil seitlich offen und umschließt das Führungselement 2 im zusammengesetzten Zustand daher nur teilweise. Der Schlitten kann somit in seitlicher Richtung, d.h. quer zur Längsachse 14 auf die Führungsschiene 1 aufgesetzt werden bzw. abgenommen werden. Lediglich an einem kurzen Abschnitt, am oberen Ende des Grundkörpers 16, befindet sich ein Bügel 20, der das Führungselement 2 vollständig umschließt. Wahlweise können auch andere Mittel vorgesehen sein, die den Schlitten 11 in Querrichtung auf der Führungsschiene 1 halten.

Der Schlitten 11 umfasst ferner Mittel zum Befestigen des Handstücks 15 des Knochenbohrers. Diese Befestigungsmittel umfassen hier einen seitlich vom Grundkörper 16 wegragenden Arm 18, sowie ein weiteres Befestigungselement 12. Der Arm 18 ist einstückig mit dem Schlitten-Grundkörper 16 gebildet. Das Befestigungselement 12 ist hier ein separates Teil.

Der Arm 18 hat eine Öffnung 25, die mit der Bohrachse 19 fluchtet. Das Befestigungselement 12 ist hier als eine Schelle ausgebildet, die an einem Schaft des Handstücks 15 montiert wird. Die Schelle 12 umfasst zwei Teile 17, 23, die mittels Schrauben 14 miteinander verschraubt werden und das Handstück 15 dazwischen festklemmen. Ein unterer Teil 17 der Schelle 12, der auf Seiten des Spiralbohrers 28 angeordnet ist, umfasst ebenfalls eine Öffnung 26, die mit der Öffnung 25 des Armes 18 fluchtet. Die Bohrachse 19 bildet dabei eine Schwenkachse, um die das Handstück 15 relativ zum Schlitten-Grundkörper 16 schwenkbar ist.

Der Schlitten 11 ist vorzugsweise gegenüber der Führungsschiene 1 nicht schwenkbar, sondern lediglich in der Bohrrichtung verschiebbar.

Im Grundkörper 16 ist ferner eine Aufnahme 29 für einen Bohrtiefenbegrenzer 22 vorgesehen. Die Aufnahme 29 umfasst hier zwei Führungskanäle, die auf beiden Seiten der zentralen Führung 13 parallel angeordnet sind und jeweils einen Schenkel des Bohrtiefenbegrenzers 22 gleitend aufnehmen. Um den Bohrtiefenbegrenzer 22 in einer gewünschten Stellung zu fixieren sind zwei Fixierschrauben 27 vorgesehen. Die beiden Schrauben 27 verlaufen jeweils durch eine im Grundkörper 16 vorgesehene Gewindebohrung und klemmen den zugehörigen Schenkel des Bohrtiefenbegrenzers 22 fest, wenn sie zugeschraubt werden.

Fig. 2c zeigt eine Ansicht des Tiefenbegrenzers von unten. Wie zu erkennen ist, umschließt der Bohrtiefenbegrenzer 22 den Spiralbohrer 28 etwa hufeisenförmig. Der Bohrtiefenbegrenzer besteht hier aus zwei separaten Teilen, kann aber auch einteilig gebildet sein. Wahlweise können beide Teile oder auch nur ein Teil des Bohrtiefenbegrenzers 22 verwendet werden.

Sämtliche Bestandteile der Führungsvorrichtung sind vorzugsweise aus rostfreiem Edelstahl hergestellt.

## Patentansprüche

1. Führungsschiene (1) für einen Knochenbohrer
**gekennzeichnet durch**
- ein Führungselement (2) zum Führen eines Schlittens (11), und
- Befestigungsmittel (3) zum Befestigen der Führungsschiene (1).

2. Führungsschiene (1) nach Anspruch 1
**dadurch gekennzeichnet,**
**dass** das Führungselement (2) ein lang gestrecktes, stabförmiges Element ist.

3. Führungsschiene (1) nach Anspruch 1 oder 2
**dadurch gekennzeichnet,**
**dass** Mittel (4) zum Abhalten des oralen mukoperiostalen Lappens im Mundraum des Patienten vorgesehen sind.

4. Führungsschiene (1) nach Anspruch 3,
**dadurch gekennzeichnet,**
**dass** sich die Mittel (4) quer zu einer Längsrichtung (14) des Führungselements (2) erstrecken und seitlich vom Führungselement (2) wegragen.

5. Führungsschiene (1) nach einem der vorhergehenden Ansprüche
**dadurch gekennzeichnet,**
**dass** die Befestigungsmittel (3) einen Arm (5) umfassen, der sich quer zu einer Längsrichtung des Führungselements (2) erstreckt und von diesem seitlich wegragt.

6. Führungsschiene (1) nach Anspruch 3 oder 4,
**dadurch gekennzeichnet,**
**dass** die Mittel (4) und die Befestigungsmittel (3) etwa in entgegengesetzte Richtungen vom Führungselement (2) wegragen.

7. Schlitten (11) für einen Knochenbohrer
**gekennzeichnet durch**
- einen Grundkörper (16) mit Mitteln (13) zum Eingreifen mit einer Führungsschiene (1) und
- Befestigungsmittel (12,18) zum Befestigen des Schlittens (11) an einem Handstück (15) des Knochenbohrers.

8. Schlitten (11) für einen Knochenbohrer nach Anspruch 7,
**dadurch gekennzeichnet,**
**dass** sich die Befestigungsmittel (12,18) im Wesentlichen quer zu einer Bohrrichtung (19) erstrecken.

9. Schlitten (11) für einen Knochenbohrer nach Anspruch 7 oder 8,
**dadurch gekennzeichnet,**
**dass** die Befestigungsmittel (12,18) derart ausgebildet sind, dass das Handstück (15) relativ zum Schlitten (11) geschwenkt werden kann.

10. Schlitten (11) für einen Knochenbohrer nach Anspruch 9,
**dadurch gekennzeichnet,**
**dass** die Befestigungsmittel (12,18) einen vom Grundkörper (16) seitlich wegragenden Arm (18) umfassen, in dem eine Öffnung (25) vorgesehen ist.

11. Schlitten (11) für einen Knochenbohrer nach einem der Ansprüche 7 bis 10,
**dadurch gekennzeichnet,**
**dass** die Befestigungsmittel (12,18) ein weiteres Befestigungselement (12) umfassen.

12. Schlitten (11) für einen Knochenbohrer nach Anspruch 11,
**dadurch gekennzeichnet,**
**dass** das Befestigungselement (12) eine Öffnung (26) aufweist, die im montierten Zustand mit der Öffnung (25) des Armes (18) fluchtet, so dass das Befestigungselement (12) relativ zum Grundkörper (16) des Schlittens (11) schwenkbar ist.

13. Schlitten (11) für einen Knochenbohrer nach einem der Ansprüche 7 bis 12,
**dadurch gekennzeichnet,**
**dass** der Schlitten (11) einen Tiefenbegrenzer (22) zum Begrenzen der Bohrtiefe aufweist.

14. Schlitten (11) für einen Knochenbohrer nach einem der Ansprüche 7 bis 13,
**dadurch gekennzeichnet,**
**dass** die Führung (13) als Führungsrille ausgebildet ist.

15. Vorrichtung zum Führen eine Knochenbohrers, umfassend eine Führungsschiene (1) nach einem der Ansprüche 1 bis 6 und einen Schlitten (11) nach einem der Ansprüche 7 bis 14.
